# EUROPÄISCHE PATENTSCHRIFT

(11) **EP 0 772 588 B1**
(45) Veröffentlichungstag und Bekanntmachung des Hinweises auf die Patenterteilung: **23.12.1998**
(21) Anmeldenummer: 95926919.2
(22) Anmeldetag: 18.07.1995
(51) Int. Cl.: C07C 269/04, C07C 271/12, C07D 295/20

(54) **HERSTELLUNG VON CARBAMINSÄUREVINYLESTERN**
PRODUCTION OF CARBAMIC ACID VINYL ESTERS
PREPARATION D'ESTERS VINYLIQUES D'ACIDE CARBAMIQUE

(30) Priorität: 20.07.1994 DE 4425677
(43) Veröffentlichungstag der Anmeldung: 14.05.1997
(73) Patentinhaber: BASF AKTIENGESELLSCHAFT, 67056 Ludwigshafen (DE)
(72) Erfinder: HEIDER, Marc, D-67433 Neustadt (DE); HENKELMANN, Jochem, D-68165 Mannheim (DE); KARCHER, Michael, D-68723 Schwetzingen (DE); RÜHL, Thomas, D-67227 Frankenthal (DE)
(74) Vertreter: Isenbruck, Günter, Dr.
(86) Internationale Anmeldenummer: EP9502805
(87) Internationale Veröffentlichungsnummer: WO9602498

(56) Entgegenhaltungen:
- FR-A- 2 576 594
- JOURNAL OF ORGANIC CHEMISTRY., Bd.54, Nr.7, 31. März 1989, EASTON US Seiten 1518 - 23 R. MAHE ET. AL. 'Catalytic Synthesis of Vinyl Carbamates from Carbon Dioxide and Alkynes with Ruthenium Complexes.'
- CHEMICAL ABSTRACTS, vol. 114, no. 20, 20. Mai 1991, Columbus, Ohio, US; abstract no. 186109t, Y. SASAKI ET. AL. 'Fixation of Carbom Dioxide Catalysed by Transition Metal Complexes. Part 3. Synthesis of Vinyl Carbamate.' Seite 2 ;Spalte 1 ; & KOGAI SHIGEN KENKYUSHO IHO, Bd.17, Nr.4, 1988 Seiten 21 - 5

## Beschreibung

Die vorliegende Erfindung bezieht sich auf ein Verfahren zur Herstellung von Vinylestern von Carbaminsäureverbindungen. Insbesondere bezieht sich die vorliegende Erfindung auf ein Verfahren zur Herstellung derartiger Verbindungen, bei der die Umsetzung von sekundären Aminen mit Kohlendioxid und Acetylenverbindungen in Gegenwart von Katalysatoren der Platinmetall-Gruppe durchgeführt wird.

Die Herstellung von Carbaminsäurevinylestern aus Vinylchlorformiat und Aminen ist eine ausführlich beschriebene Reaktion (R.A. Olofson et al; THL 18, 1563 - 1566 (1977)).

Zur Herstellung des Vinylchlorformiates sind ausgehend von Kohlenoxychlorid zwei Varianten bekannt. Zum einen die Reaktion mit Quecksilberenolat (R. A. Olofson et al; JOC, 43, 752, (1978)), zum anderen die Umsetzung mit Ethylenglykol zu Ethylenglykol-bis-chlorformiat, das im Anschluß thermisch zum Vinylchlorformiat gespalten wird (L. H. Lee, JOC 30, 3943, (1965); U.S. Patent 2,377,085).

Neben den erforderlichen hohen Sicherheitsmaßnahmen beim Arbeiten mit Kohlenoxychlorid birgt die Verwendung von Quecksilber-organischen Verbindungen ein hochtoxisches Risiko.

Die thermische Eliminierung des Ethylenglykol-bis-chlorformiats gelingt nur mit schlechter Selektivität und Bildung von toxikologisch bedenklichen Nebenprodukten.

Von P. H. Dixneuf wurde eine Synthese von Carbaminsäurevinylestern aus sekundären Aminen, Kohlendiox id und Acetylen beschrieben (J. Org. Chem. 54, 1518 (1989)). Dabei wurde Acetylen bei -50 °C in einem Lösungsmittel gelöst und in einem Autoklaven mit Kohlendioxid und einem sekundären Amin unter Rutheniumtrichloridkatalyse bei 80 °C zum Carbaminsäurevinylester umgesetzt. Die Ausbeuten bei diesem Prozeß sind mäßig.

J. of Organic Chemistry, Bd. 54, no. 7, S. 1518 (1989) betrifft ein Verfahren zur Herstellung von Carbaminsäurevinylestern durch Umsetzung von sekundärem Amin mit Kohlendioxid und Acetylen, Vinylacetylen oder Hexin, wobei in Gegenwart von Rutheniumverbindungen und ggf. katalytischen Mengen eines freien Phosphins Vinylcarbamate erhalten werden.

Der Erfindung liegt die Aufgabe zugrunde, das aus dem Stand der Technik bekannte Verfahren dahingehend zu verbessern, daß hohe Ausbeuten und/oder hohe Selektivität, vorzugsweise beides, erreicht wird.

Diese Aufgabe wird erfindungsgemäß durch ein Verfahren zur Herstellung von Carbaminsäurevinylester-Verbindungen mit den nachfolgenden Merkmalen gelößt. Diese Verbindungen sind durch die Formel (I) bzw. (II) gekennzeichnet 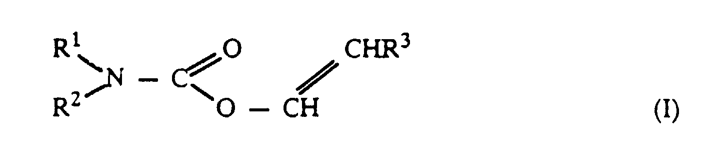 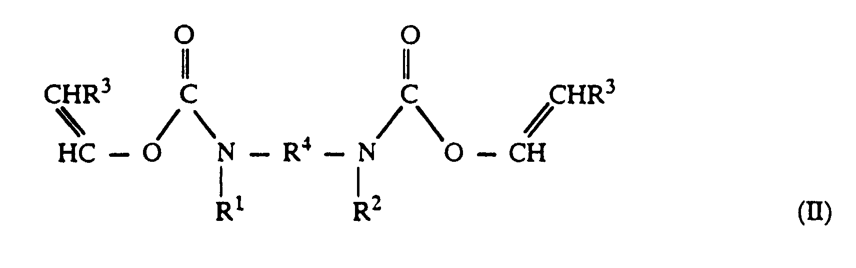 in denen
R¹ und R² jeweils
   einwertige Kohlenwasserstoffreste mit 1 bis 6 C-Atomen, insbesondere Alkyl oder mit Aryl und/oder Cycloalkyl substituiertes Alkyl mit 1 bis 6 C-Atomen,
   Aryl oder mit Alkyl und/oder Cycloalkyl substituiertes Aryl mit 6 bis 10 C-Atomen und
   Cycloalkyl oder mit Alkyl und/oder Aryl substituiertes Cycloalkyl mit 3 bis 10 C-Atomen bedeuten, oder
R¹ und R² zusammen mit dem Stickstoff, an den sie gebunden sind, eine heterocyclische Gruppe mit 5 bis 7 Ringgliedern und 1 bis 3 Heteroatomen aus der Gruppe O, N, S bilden,
R³ Alkyl oder Aralkyl mit 1 bis 6 C-Atomen,
   Cycloalkyl mit 3 bis 10 C-Atomen,
   Aryl oder Alkaryl mit 6 bis 10 C-Atomen bedeutet,
R⁴ zweiwertige Kohlenwasserstoffreste mit 1 bis 10 C-Atomen, insbesondere zweiwertiges Alkyl oder mit Aryl und/oder Cycloalkyl substituiertes Alkyl mit 1 bis 6 C-Atomen,
   zweiwertiges Aryl oder mit Alkyl und/oder Cycloalkyl substituiertes Aryl mit 6 bis 10 C-Atomen und
   zweiwertiges Cycloalkyl oder mit Alkyl und/oder Aryl substituiertes Cycloalkyl mit 3 bis 10 C-Atomen bedeutet, oder
R⁴ und R¹ und/oder R⁴ und R² oder R⁴ und R² und R¹ mit den jeweiligen Stickstoffatomen, an die sie gebunden sind,
eine heterocyclische Gruppe mit 5 bis 7 Ringgliedern und 1 bis 3 Heteroatomen aus der Gruppe O, N, S bilden.

Bei dem Verfahren werden die entsprechenden sekundären Amine der Formeln (III) und (IV) 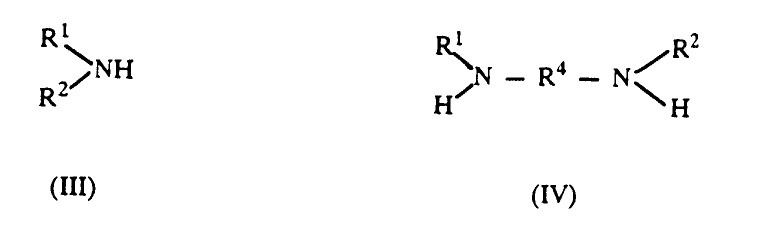 in denen R¹, R², R³ und R⁴ die oben angegebenen Bedeutungen haben, mit Kohlendioxid und acetylenisch ungesättigten Verbindungen der Formel (V)

R³ ― C ≡ CH (V)

in der R³ die oben angegebene Bedeutung hat, umgesetzt. Diese Umsetzung erfolgt in Gegenwart einer Verbindung eines Metalls aus der Platingruppe, insbesondere einer Rutheniumverbindung.

Erfindungsgemäß wird bei dieser Reaktion der Verfahrensschritt (b) und wahlweise (a), vorzugsweise beide, durchgeführt:
(a) während der Umsetzung wird die Acetylenverbindung nachgepreßt;
(b) die Umsetzung wird in Gegenwart eines tertiären Amins durchgeführt.

Überraschenderweise wurde gefunden, daß sich sekundäre Amine mit Kohlendioxid und Acetylen mit deutlich höheren Ausbeuten zu den Carbaminsäurevinylestern umsetzen lassen, wenn man Acetylen während der Reaktion ständig nachpreßt und ein tertiäres Amin der Reaktion zusetzt.

### Aminverbindungen

Als Aminverbindungen der Formeln (III) und (IV) eignen sich insbesondere
Piperidinverbindungen
Morpholinverbindungen
Piperazinverbindungen
Dialkylaminverbindungen mit 2 bis 20 C-Atomen.

Bevorzugte Aminverbindungen sind Piperidin, N-Ethylpiperidin, Morpholin oder Diethylamin.

### Acetylenverbindungen

Bevorzugte Beispiele der erfindungsgemäß einsetzbaren Acetylenverbindungen sind
Acetylen
Propin
1-Butin
1-Hexin
Phenylacetylen
Vinylacetylen
Diacetylen

### Katalysatoren

Als Katalysatoren können Verbindungen der Platinmetalle in allen Oxidationsstufen, bevorzugt Verbindungen des Rutheniums und des Palladiums als solche, z. B. RuCl₃, Ru(acac)₃, Ru₃(CO)₁₂ oder PdCl₂, eingesetzt werden. Verschiedene Komplexbildner können für die Verbindungen der Metalle der Platingruppe eingesetzt werden. Bevorzugt sind aliphatische und aromatische Phosphine, Phosphite und Amine (z. B. Tributylphosphin, Triphenylphosphin, Trimethylphosphit, Triethylamin). Auch die Verwendung mehrzähniger Liganden wie 1,2-Bis(dimethylphosphino)ethan oder Tetramethylethylendiamin (TMEDA) ist erfindungsgemäß möglich.

Die Platinmetallverbindung wird in Mengen von 0,1 bis 4 mol-%, bevorzugt zwischen 0,5 und 2 mol-%, bezogen auf eingesetzte Amingruppe, eingesetzt. Der Komplexbildner wird vorzugsweise in 0,5- bis 4facher molarer Menge, bezogen auf die eingesetzte Platinmetallverbindung, eingesetzt.

### Bevorzugte Betriebsbedingungen

Die Reaktion wird zwischen 50 und 200 °C, insbesondere zwischen 80 und 120 °C, durchgeführt. Der Druck der Reaktion liegt zwischen 1 und 50 bar. Die Reaktionsdauer beträgt 1 bis 12 Stunden.

Besonders vorteilhaft ist es, Kohlendioxid zuerst mit dem Amin umzusetzen und erst danach die Acetylenverbindung zur Reaktion zu geben.

Das Amin kann als solches, falls es flüssig ist, oder aber als Lösung in verschiedenen organischen Lösungsmitteln wie Toluol, Tetrahydrofuran oder Acetonitril eingesetzt werden.

### Tertiäre Amine

Die erfindungsgemäß eingesetzten tertiären Amine sind vorzugsweise Trihydrocarbylamine mit 3 bis 30 C-Atomen. Beispiele für die einsetzbaren tertiären Amine sind
Triethylamin
4-Dimethylaminopyridin
DABCO (Diazabicyclooctan)
1,8-Bis(dimethylamino)-naphthalin
Trioctylamin.

Triethylamin ist gegenwärtig bevorzugt.

Die Trihydrocarbylamine werden dabei in katalytischen bis stöchiometrischen Mengen eingesetzt, wobei sich stöchiometrische Mengen auf das Mol von sekundärer Amingruppe der eingesetzten Verbindungen der Formel (III) oder (IV) beziehen.

### Gewinnung der Carbaminsäurevinylester-Verbindungen

Die in dem erfindungsgemäßen Verfahren gebildeten Carbaminsäurevinylester-Verbindungen werden aus dem Reaktionsgemisch vorzugsweise durch einfache Destillation des rohen Vinylierungsproduktes gewonnen.

Bevorzugte Ausführungsformen der Erfindung sind in den Unteransprüchen und in den folgenden Beispielen angegeben.

### Beispiel 1

Zu 75 g Acetonitril wurden 75 g Piperidin und 1,5 g Rutheniumtrichlorid-Hydrat gegeben und bei Raumtemperatur 10 bar Kohlendioxid bis zur Druckkonstanz aufgepreßt. Danach wurde der Ansatz auf 100 °C erhitzt und Acetylen bis zu einem Gesamtdruck von 20 bar nachgepreßt, bis der Druck konstant war.

Anschließend wurde der Reaktionsansatz mit wäßriger Salzsäure gewaschen, das Acetonitril entfernt und destilliert.

Die Ausbeute (mol% auf eingesetztes sek. Amin) an N-Piperidincarbonsäurevinylester betrug 63% der Theorie.

### Beispiel 2

Analog Beispiel 1 wurde Morpholin mit Kohlendioxid und Acetylen umgesetzt. Nach Aufarbeitung und Destillation betrug die Ausbeute an N-Morpholincarbonsäurevinylester 49% der Theorie.

### Beispiel 3

Analog Beispiel 1 wurde N-Ethyl-piperazin mit Kohlendioxid und Acetylen umgesetzt. Nach Entfernung des Acetonitrils wurde das Produkt destilliert. Die Ausbeute an N-Ethyl-piperazin-N'-carbonsäurevinylester betrug 93% der Theorie.

### Beispiel 4

Analog Beispiel 1 wurde Diethylamin mit Kohlendioxid und Acetylen umgesetzt. Nach Entfernung des Acetonitrils und Destillation betrug die Ausbeute an N,N-Diethylcarbaminsäurevinylester 10% der Theorie.

### Beispiel 5

Analog Beispiel 1 wurde Diethylamin in Acetonitril und 30 ml Triethylamin mit Kohlendioxid und Acetylen umgesetzt. Die Ausbeute an N,N-Diethylcarbaminsäurevinylester betrug nach Aufarbeitung und Destillation 75% der Theorie.

## Patentansprüche

1. Verfahren zur Herstellung von Carbaminsäurevinylester-Verbindungen der Formel (I) oder (II) 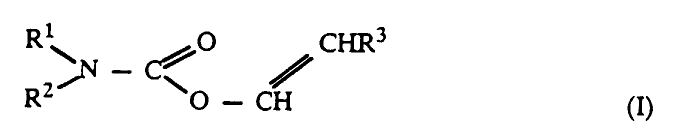 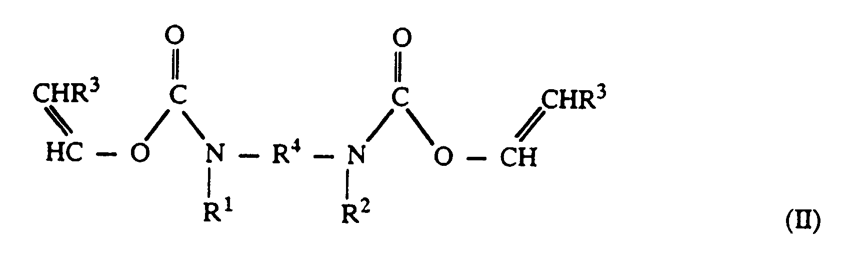 in denen
R¹ und R² jeweils
einwertige Kohlenwasserstoffreste mit 1 bis 6 C-Atomen, insbesondere
Alkyl oder mit Aryl und/oder Cycloalkyl substituiertes Alkyl mit 1 bis 6 C-Atomen,
Aryl oder mit Alkyl und/oder Cycloalkyl substituiertes Aryl mit 6 bis 10 C-Atomen und
Cycloalkyl oder mit Alkyl und/oder Aryl substituiertes Cycloalkyl mit 3 bis 10 C-Atomen bedeuten, oder
R¹ und R² zusammen mit dem Stickstoff, an den sie gebunden sind, eine heterocyclische Gruppe mit 5 bis 7 Ringgliedern und 1 bis 3 Heteroatomen aus der Gruppe O, N, S bilden,
R³ Alkyl oder Aralkyl mit 1 bis 6 C-Atomen,
Cycloalkyl mit 3 bis 10 C-Atomen,
Aryl oder Alkaryl mit 6 bis 10 C-Atomen bedeutet,
R⁴ zweiwertige Kohlenwasserstoffreste mit 1 bis 10 C-Atomen, insbesondere
zweiwertiges Alkyl oder mit Aryl und/oder Cycloalkyl substituiertes Alkyl mit 1 bis 6 C-Atomen,
zweiwertiges Aryl oder mit Alkyl und/oder Cycloalkyl substituiertes Aryl mit 6 bis 10 C-Atomen und
zweiwertiges Cycloalkyl oder mit Alkyl und/oder Aryl substituiertes Cycloalkyl mit 3 bis 10 C-Atomen bedeutet, oder
R⁴ und R¹ und/oder R⁴ und R² oder R⁴ und R² und R¹ mit den jeweiligen Stickstoffatomen, an die sie gebunden sind,
eine heterocyclische Gruppe mit 5 bis 7 Ringgliedern und 1 bis 3 Heteroatomen aus der Gruppe O, N, S bilden,
durch Umsetzen von entsprechenden sekundären Aminen der Formeln (III) und (IV) 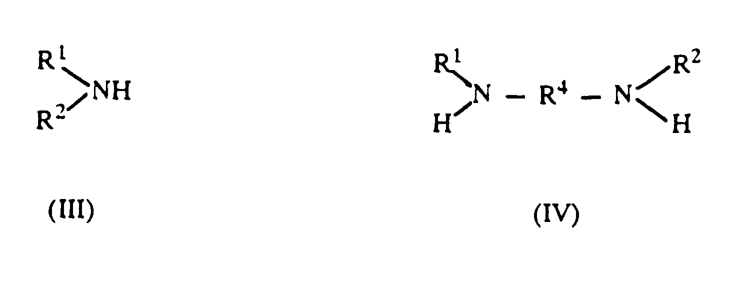 mit Kohlendioxid und acetylenisch ungesättigten Verbindungen der Formel (V)
R³ ― C ≡ CH (V)
in der R³ die oben angegebene Bedeutung hat,
in Gegenwart einer Verbindung eines Metalls aus der Platingruppe, insbesondere einer Rutheniumverbindung,
dadurch gekennzeichnet, daß
man den Verfahrensschritt (b) und wahlweise (a), vorzugsweise beide, durchführt:
(a) während der Umsetzung wird die Acetylenverbindung nachgepreßt;
(b) die Umsetzung wird in Gegenwart eines tertiären Amins durch geführt.

2. Verfahren nach Anspruch 1, dadurch gekennzeichnet, daß die Reaktion unter folgenden Bedingungen durchgeführt wird:
| | |
|---|---|
| Temperatur | 50 bis 200 °C |
| Druck | 1 bis 50 bar |
| Dauer | 1 bis 12 Stunden |

3. Verfahren nach Anspruch 1, dadurch gekennzeichnet, daß man die Umsetzung des Amins mit Kohlendioxid und Acetylen in Gegenwart von Ru^{III}-Verbindungen durchführt.

4. Verfahren nach Anspruch 3, dadurch gekennzeichnet, daß man die Umsetzung des Amins mit Kohlendioxid und Acetylenverbindung in Gegenwart von Rutheniumtrichlorid als Katalysator durchführt.

5. Verfahren nach einem der vorhergehenden Ansprüche, dadurch gekennzeichnet, daß man die Umsetzung des Amins mit Kohlendioxid und Acetylenverbindung in Gegenwart von Verbindungen der Platinmetalle unter Zusatz von Komplexbildnern, ausgewählt aus Phosphinen, Phosphiten und Aminen, durchführt.

6. Verfahren nach einem der vorhergehenden Ansprüche, dadurch gekennzeichnet, daß man als Verbindung der Formel (III) eine Piperidinverbindung, eine Morpholinverbindung, eine Piperazinverbindung oder eine Dialkylaminverbindung, vorzugsweise Piperidin, Morpholin oder Diethylamin, verwendet.

7. Verfahren nach einem der vorhergehenden Ansprüche, dadurch gekennzeichnet, daß man in einem Lösungsmittel arbeitet.

8. Verfahren nach Anspruch 7, dadurch gekennzeichnet, daß man als Lösungsmittel eines aus der Gruppe
Toluol
Tetrahydrofuran
Acetonitril
DMSO (Dimethylsulfoxid)
NMP (N-Methylpyrrolidon),
vorzugsweise Acetonitril,
verwendet.

9. Verfahren nach einem der vorhergehenden Ansprüche, dadurch gekennzeichnet, daß man mit einem Überschuß an Acetylenverbindung von 1,5 mol bis 10 mol Acetylenverbindung bezogen auf 1 mol eingesetzte reaktionsfähige Amingruppe arbeitet.

10. Verfahren nach einem der vorhergehenden Ansprüche, dadurch gekennzeichnet, daß man als Acetylenverbindung Acetylen (R³ = H) verwendet.

11. Verfahren nach einem der vorhergehenden Ansprüche, dadurch gekennzeichnet, daß man sowohl die Acetylenverbindung während der Reaktion nachpreßt als auch in Gegenwart von tertiärem Amin arbeitet.

12. Verfahren nach einem der vorhergehenden Ansprüche, dadurch gekennzeichnet, daß man als tertiäres Amin Triethylamin verwendet.

13. Verfahren nach einem der vorhergehenden Ansprüche, dadurch gekennzeichnet, daß man zunächst die sekundäre Aminverbindung mit Kohlendioxid umsetzt und anschließend die Acetylenverbindung zusetzt.

14. Verfahren nach Anspruch 14, dadurch gekennzeichnet, daß in einer ersten Stufe 1 - 2 mol CO₂ mit der sekundären Aminverbindung umsetzt, vorzugsweise bei etwa Zimmertemperatur, einem Druck von etwa 50 bar CO₂, und einer Dauer von 0,5 bis 4 Stunden (im Fälle von Druckkonstanz) und daß man in einer zweiten Stufe, bezogen auf eingesetzte reaktive Aminverbindung, 1 - 10 mol der Acetylenverbindung, insbesondere C₂H₂, zugibt und zur Umsetzung bringt, vorzugsweise bei einer Temperatur von 80 - 120 °C und einer Dauer von 2 bis 12 Stunden (im Falle von Druckkonstanz).

## Claims

1. A process for preparing vinyl carbamate compounds of the formula (I) or (II) 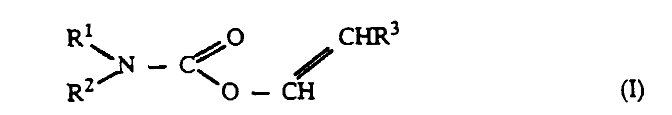 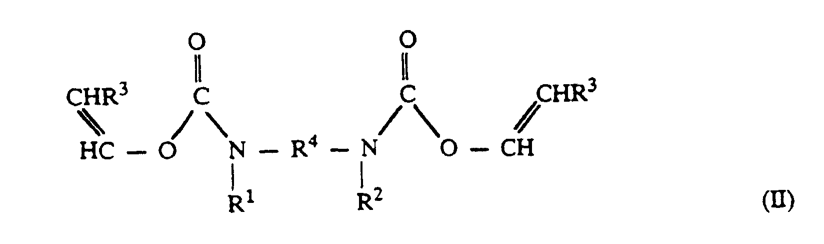 where
R¹ and R² are each
monovalent hydrocarbon radicals with 1 to 6 carbon atoms, in particular
alkyl or aryl- and/or cycloalkyl-substituted alkyl with 1 to 6 carbon atoms,
aryl or alkyl- and/or cycloalkyl-substituted aryl with 6 to 10 carbon atoms and
cycloalkyl or alkyl- and/or aryl-substituted cycloalkyl with 3 to 10 carbon atoms, or
R¹ and R² form, together with the nitrogen to which they are bonded, a heterocyclic group with 5 to 7 ring members and 1 to 3 hetero atoms from the group of O, N, S,
R³ is alkyl or aralkyl with 1 to 6 carbon atoms, cycloalkyl with 3 to 10 carbon atoms, aryl or alkaryl with 6 to 10 carbon atoms,
R⁴ is a divalent hydrocarbon radical with 1 to 10 carbon atoms, in particular alkylene or aryl- and/or cycloalkyl-substituted arylene with 6 to 10 carbon atoms and cycloalkylene or alkyl- and/or aryl-substituted cycloalkylene with 3 to 10 carbon atoms, or
R⁴ and R¹ and/or R⁴ and R² or R⁴ and R² and R¹ form, with the particular nitrogen atoms to which they are bonded,
a heterocyclic group with 5 to 7 ring members and 1 to 3 hetero atoms from the group of O, N, S,
by reacting appropriate secondary amines of the formulae (III) and (IV) 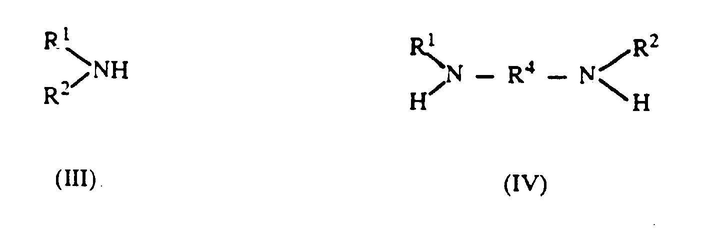 with carbon dioxide and acetylenically unsaturated compounds of the formula (V)
R³-C≡CH (V)
where R³ has the abovementioned meaning,
in the presence of a compound of a metal from the platinum group, in particular a ruthenium compound, wherein the two process step (b) with or without (a), preferably both, are carried out:
(a) the acetylene compound is reinjected during the reaction;
(b) the reaction is carried out in the presence of a tertiary amine.

2. A process as claimed in claim 1, wherein the reaction is carried out under the following conditions:
| | |
|---|---|
| temperature | 50 to 200°C |
| pressure | 1 to 50 bar |
| time | 1 to 12 hours |

3. A process as claimed in claim 1, wherein the reaction of the amine with carbon dioxide and acetylene is carried out in the presence of Ru^{III} compounds.

4. A process as claimed in claim 3, wherein the reaction of the amine with carbon dioxide and acetylene compound is carried out in the presence of ruthenium trichloride as catalyst.

5. A process as claimed in any of the preceding claims, wherein the reaction of the amine with carbon dioxide and acetylene compound is carried out in the presence of compounds of the platinum metals with the addition of complexing agents selected from phosphines, phosphites and amines.

6. A process as claimed in any of the preceding claims, wherein a piperidine compound, a morpholine compound, a piperazine compound or a dialkylamine compound, preferably piperidine, morpholine or diethylamine, is used as compound of the formula (III).

7. A process as claimed in any of the preceding claims, wherein a solvent is used.

8. A process as claimed in claim 7, wherein the solvent used is one from the group of
toluene
tetrahydrofuran
acetonitrile
DMSO (dimethyl sulfoxide)
NMP (N-methylpyrrolidone),
preferably acetonitrile.

9. A process as claimed in any of the preceding claims, wherein an excess of acetylene compound of from 1.5 mol to 10 mol of acetylene compound based on 1 mol of reactive amino group employed is used.

10. A process as claimed in any of the preceding claims, wherein acetylene (R³ = H) is used as acetylene compound.

11. A process as claimed in any of the preceding claims, wherein both the acetylene compound is reinjected during the reaction and a tertiary amine is present.

12. A process as claimed in any of the preceding claims, wherein triethylamine is used as tertiary amine.

13. A process as claimed in any of the preceding claims, wherein first the secondary amine compound is reacted with carbon dioxide and subsequently the acetylene compound is added.

14. A process as claimed in claim 13, wherein in a first stage 1 - 2 mol of CO₂ are reacted with the secondary amine compound, preferably at about room temperature, under a pressure of about 50 bar of CO₂, and for a time of from 0.5 to 4 hours (in the case of constant pressure), and in a second stage 1 - 10 mol of the acetylene compound, in particular C₂H₂, are added and reacted, based on reactive amine compound employed, preferably at 80 - 120°C for 2 to 12 hours (in the case of constant pressure).

## Revendications

1. Procédé de préparation de composés ester vinylique d'acide carbamique de formule (I) ou (II) 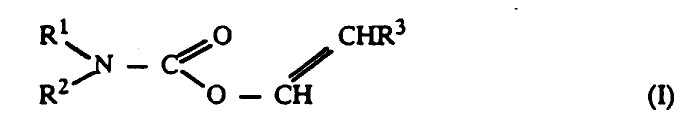 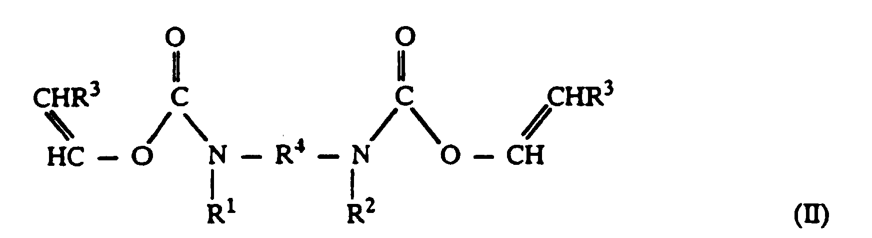 dans lesquels
R¹ et R² représentent chacun
des restes hydrocarbonés monovalents ayant 1-6 atomes de carbone, en particulier
des groupements alkyle ou alkyle à 1-6 atomes de carbone substitués par des groupements aryle et/ou cycloalkyle,
des groupements aryle ou aryle à 6-10 atomes de carbone substitués par des groupements alkyle et/ou cycloalkyle,
des groupements cycloalkyle ou cycloalkyle à 3-10 atomes de carbone substitués par des groupements alkyle et/ou aryle, ou bien
R¹ et R² représentent ensemble, avec l'atome d'azote qui les relie, un groupement hétérocyclique à 5-7 maillons dans le cycle et comportant 1 à 3 hétéroatomes choisis parmi O, N et S,
R³ représente un groupement alkyle ou aralkyle à 1-6 atomes de carbone, un groupement cycloalkyle à 3-10 atomes de carbone, un groupement aryle ou alkaryle à 6-10 atomes de carbone,
R⁴ représente un reste hydrocarboné bivalent à 1-10 atomes de carbone, en particulier
un groupement alkyle bivalent ou un groupement alkyle à 1-6 atomes de carbone substitué par des groupements aryle et/ou cycloalkyle,
un groupement aryle bivalent ou un groupement aryle à 6-10 atomes de carbone substitué par des groupements alkyle et/ou cycloalkyle, et
un groupement cycloalkyle bivalent ou un groupement cycloalkyle à 3-10 atomes de carbone substitué par des groupements alkyle et/ou aryle, ou bien
R⁴ et R¹ et/ou R⁴ et R² ou R⁴ et R² et R¹ représentent, avec les atomes d'azote respectifs qui les relie, un groupement hétérocyclique à 5-7 maillons dans le cycle et comportant 1 à 3 hétéroatomes choisis parmi O, N et S,
par réaction des amines secondaires correspondantes de formules (III) et (IV) 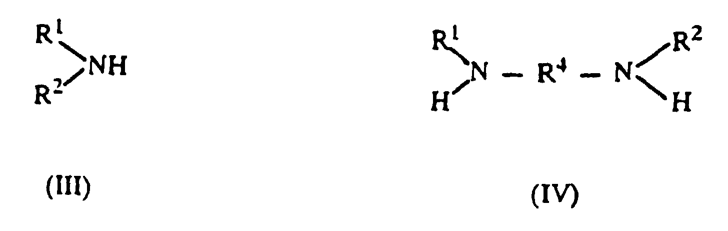 avec du dioxyde de carbone et des composés à insaturation acétylénique de formule (V)
R³ - C≡CH (V)
dans laquelle R³ prend la signification susmentionnée,
en présence d'un composé d'un métal du groupe du platine, en particulier d'un composé du ruthénium, caractérisé en ce que l'on effectue les étapes de procédé (b) et éventuellement (a), de préférence les deux avec :
(a) le composé acétylénique est recomprimé pendant la réaction ;
(b) la réaction est menée en présence d'une amine tertiaire.

2. Procédé selon la revendication 1, caractérisé en ce que la réaction est menée dans les conditions réactionnelles suivantes :
| | |
|---|---|
| Température | 50-200°C |
| Pression | 1-50 bar |
| Durée | 1-12 heures. |

3. Procédé selon la revendication 1, caractérisé en ce que la réaction de l'amine avec le dioxyde de carbone et l'acétylène est menée en présence de composés du Ru^{III}.

4. Procédé selon la revendication 3, caractérisé en ce que la réaction de l'amine avec le dioxyde de carbone et le composé acétylénique est menée en présence de trichlorure de ruthénium, en tant que catalyseur.

5. Procédé selon l'une quelconque des revendications 1 à 4, caractérisé en ce que la réaction de l'amine avec le dioxyde de carbone et le composé acétylénique est menée en présence de composés de métaux du groupe du platine avec ajout d'agents complexants choisis parmi les phosphines, les phosphites et les amines.

6. Procédé selon l'une quelconque des revendications 1 à 5, caractérisé en ce que l'on utilise, en tant que composé de formule (III), un composé de la pipéridine, de la morpholine, de la pipérazine ou un composé dialkylaminé, de préférence la pipéridine, la morpholine ou la diéthylamine.

7. Procédé selon l'une quelconque des revendications 1 à 6, caractérisé en ce que l'on travaille dans un solvant.

8. Procédé selon la revendication 7, caractérisé en ce que l'on utilise, en tant que solvant, l'un des éléments choisi parmi
le toluène,
le tétrahydrofurane,
l'acétonitrile,
le DMSO (diméthylsulfoxyde),
la NMP (N-méthylpyrrolidone),
de préférence l'acétonitrile.

9. Procédé selon l'une quelconque des revendications 1 à 8, caractérisé en ce que l'on travaille avec un excès en composé acétylénique de 1,5-10 moles de composé acétylénique, pour 1 mole de groupement amine pouvant réagir.

10. Procédé selon l'une quelconque des revendications 1 à 9, caractérisé en ce que l'on utilise de l'acétylène (R³ = H) comme composé acétylénique.

11. Procédé selon l'une quelconque des revendications 1 à 10, caractérisé en ce que le composé acétylénique est recomprimé pendant la réaction et en ce que l'on travaille en présence d'une amine tertiaire.

12. Procédé selon l'une quelconque des revendications 1 à 11, caractérisé en ce que l'on utilise de la triéthylamine en tant qu'amine tertiaire.

13. Procédé selon l'une quelconque des revendications 1 à 12, caractérisé en ce que l'on fait tout d'abord réagir le composé amine secondaire avec du dioxyde de carbone et qu'on ajoute ensuite le composé acétylénique.

14. Procédé selon la revendication 13, caractérisé en ce que l'on fait réagir, dans une première étape, 1-2 moles de CO₂ avec le composé amine secondaire, de préférence à température ambiante environ, sous une pression de 50 bar de CO₂ environ, et pendant 0,5-4 heures (si la pression reste constante) et en ce que l'on ajoute, dans une deuxième étape, 1-10 moles de composé acétylénique, en particulier de C₂H₂, par rapport au composé amine réactif ajouté, et on le fait réagir, de préférence à une température de 80-120°C et pendant 2-12 heures (si la pression reste constante).
